# EUROPEAN PATENT SPECIFICATION

(11) **EP 1 210 350 B1**
(45) Date of publication and mention of the grant of the patent: **14.07.2004**
(21) Application number: 00954091.5
(22) Date of filing: 16.08.2000
(51) Int. Cl.: C07D 498/18, C08G 65/329, A61K 31/445, A61K 47/48

(54) **WATER SOLUBLE SDZ-RAD ESTERS**
WASSERLÖSLICHE SDZ-RAD ESTER
ESTERS SDZ-RAD HYDROSOLUBLES

(30) Priority: 18.08.1999 US 376685
(43) Date of publication of application: 05.06.2002
(73) Proprietor: Wyeth, Madison, New Jersey 07940-0874 (US)
(72) Inventor: ZHU, Tianmin, Monroe, NY 10950 (US); SHAH, Syed, Muzafar, East Hanover, NJ 07936 (US); SAUNDERS, Richard, William, Palisades, NY 10964 (US)
(74) Representative: Wileman, David Francis, Dr.
(86) International application number: PCT/US2000/022419
(87) International publication number: WO 2001/012633

(56) References cited:
- EP-A- 0 509 795
- EP-A- 0 649 659
- EP-A- 0 781 776
- EP-B- 0 014 263
- WO-A-92/05179
- WO-A-92/21341
- WO-A-94/09010
- US-A- 5 665 772
- ZALIPSKY S ET AL: "ATTACHMENT OF DRUGS TO POLYETHYLENE GLYCOLS" EUROPEAN POLYMER JOURNAL,GB,PERGAMON PRESS, LONDON, vol. 19, no. 12, 1983, pages 1177-1183, XP000646875 ISSN: 0014-3057
- SCHULER W. ET AL: 'SDZ RAD A NEW RAPAMYCIN DERIVATIVE' TRANSPLANTATION vol. 64, no. 1, 1997, pages 36 - 42

## Description

This invention relates to water soluble SDZ-RAD esters, methods for their preparation and to their use for the preparation of a medicament for inducing immunosuppression and for the treatment of transplantation rejection, autoimmune diseases, solid tumors. More particularly, this invention concerns pegylated esters of SDZ-RAD, methods for their preparation and to their use for the preparation of a medicament for inducing immunosuppression, and for the treatment of transplantation rejection, graft vs. host disease, autoimmune diseases, diseases of inflammation, adult T-cell leukemia/lymphoma, solid tumors, fungal infections, and hyperproliferative vascular disorders.

SDZ-RAD is 40-O-(2-hydroxy)ethyl-rapamycin, the structure and synthesis of which is disclosed in WO 94/09010 (Cottens et al.).

Authors Crowe and Lemaire describe the *in vitro* and *in situ* absorption of SDZ-RAD, an analog of rapamycin having the structure: 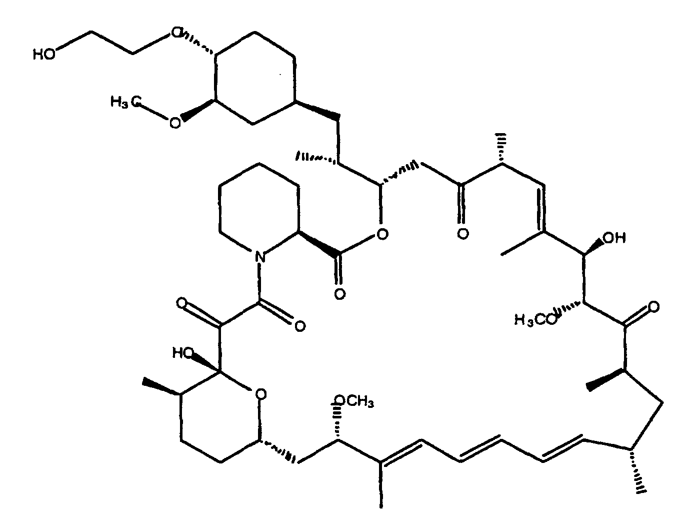 in their article in Pharmaceutical Research, Vol. 15, No. 11, 1998.

Rapamycin is a macrocyclic triene antibiotic produced by Streptomyces hygroscopicus, which was found to have antifungal activity, particularly against Candida albicans, both in vitro and in vivo [C. Vezina et al., J. Antibiot. 28, 721 (1975); S.N. Sehgal et al., J. Antibiot. 28, 727 (1975); H. A. Baker et al., J. Antibiot. 31, 539 (1978); U.S. Patent 3,929,992; and U.S. Patent 3,993,749].

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); R. Y. Calne et al., Lancet 1183 (1978); and U.S. Patent 5,100,899].

Rapamycin has also been shown to be useful in preventing or treating systemic lupus erythematosus [U.S. Patent 5,078,999], pulmonary inflammation [U.S. Patent 5,080,899], insulin dependent diabetes mellitus [Fifth Int. Conf. Inflamm. Res. Assoc. 121 (Abstract), (1990)], smooth muscle cell proliferation and intimal thickening following vascular injury [Morris, R. J. Heart Lung Transplant 11 (pt. 2): 197 (1992)], adult T-cell leukemia/lymphoma [European Patent Application 525,960 A1], and ocular inflammation [European Patent Application 532,862 A1].

Mono- and diacylated derivatives of rapamycin (esterified at the 28 and 43 positions) have been shown to be useful as antifungal agents (U.S. Patent 4,316,885) and used to make water soluble aminoacyl prodrugs of rapamycin (U.S. Patent 4,650,803). Recently, the numbering convention for rapamycin has been changed; therefore according to Chemical Abstracts nomenclature, the esters described above would be at the 31- and 42- positions. U.S. Patent 5,023,263 describes the preparation and use of 42-oxorapamycin and U.S. Patent 5,023,264 describes the preparation and use of 27-oximes of rapamycin.

Polyethylene glycol (PEG) is a linear or branched, neutral polymer available in a variety of molecular weights and is soluble in water and most organic solvents. At molecular weights less than 1000 PEGs are viscous, colorless liquids; higher molecular weight PEGs are waxy, white solids. The melting point of the solid is proportional to the molecular weight, approaching a plateau at 67°C. Molecular weights range from a few hundred to approximately 20,000 are commonly used in biological and biotechnological applications. Of much interest in the biomedical areas is the fact that PEG is nontoxic and was approved by FDA for internal consumption.

SDZ-RAD is a rapamycin derivative which has immunosuppressive activity in animal model (US patent 5,665,772).

### Description of the Invention

This invention provides novel polyethylene glycol esters of SDZ-RAD, an analog of rapamycin, which are compounds of the formula (I): 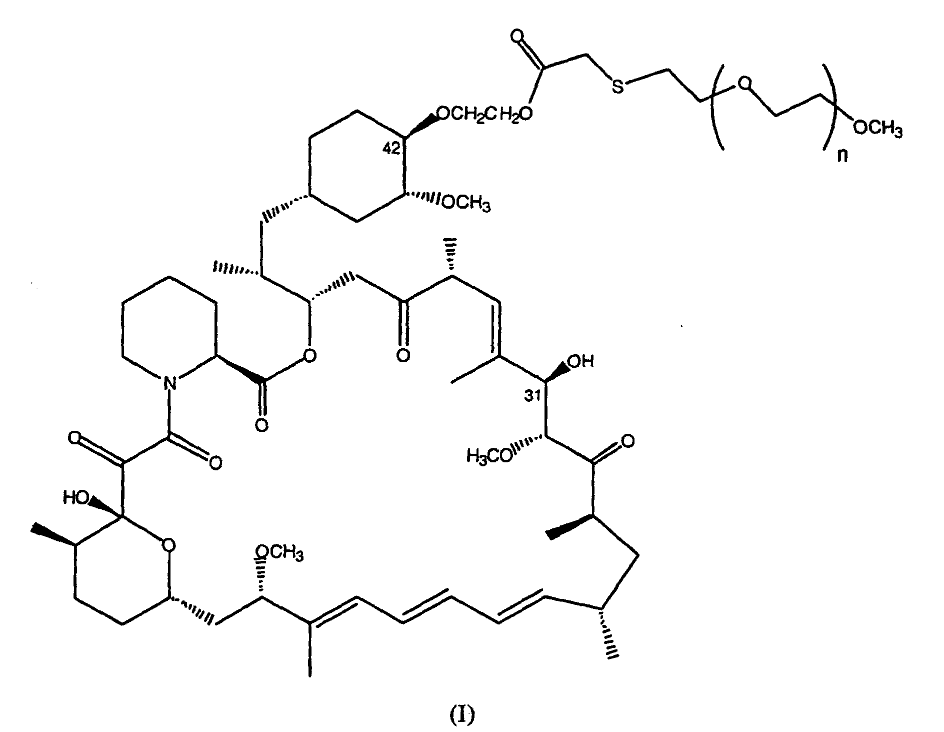 wherein n is an integer from about 5 to about 450.

The compounds of this invention are water soluble and are useful as immunosuppressive, anti inflammatory, antifungal, antiproliferative, and antitumor agents. Of the compounds of this invention, it is preferred that n = 5 - 200; more preferred that n = 8 - 135. Most preferred members are those in which n = 8 - 20 and those in which n = 90 - 120. The compounds of this invention may also be described and understood based upon the average molecular weight of the polyethylene glycol chains used to produce their ester chains. For instance, an SDZ-RAD-PEG-SH 5000 ester refers to a compound of the general formula above in which the 42-O-(2-Hydroxy)-ethyl position ester is formed utilizing a polyethylene glycol derivative having an average molecular weight range at or near 5,000.

The esters of the present invention may be produced utilizing the polyethylene glycols known in the art, such as those described on pages 355 to 361 of the Handbook of Pharmaceutical Excipients, Second Edition, 1994 (Library of Congress Catalog Card No. 94-79492), which are incorporated herein by reference. The preferred compounds of this invention may also be described as those of the formula esterified using polyethylene glycols having an average molecular weight of from about 200 to about 200,000. A preferred range of the PEG esters of this invention includes those in which the molecular weight of the polyethylene glycol portion of the ester chain has a molecular weight in the range of from about 300 to about 20,000, more preferably between about 350 and about 6,000. Non-limiting specific examples of compounds of this invention include compounds of the formula above prepared using each of the PEGs listed in the Handbook of Pharmaceutical Excipients, Second Edition, 1994.

The invention also relates to the use of compounds of formula (I) for the preparation of a medicament for treating transplantation rejection or graft vs. host disease, fungal infection, rheumatoid arthritis, restenosis, pulmonary inflammation and solid tumors in a mammal.

Because of the activity profile obtained, the compounds of this invention also are considered to have antitumor, antifungal activities, and antiproliferative activities. When used for this purpose, the compounds of this invention can be administered prior to the procedure, during the procedure, subsequent to the procedure, or any combination of the above.

When administered the compounds of this invention can be administered to a mammal orally, parenterally, intranasally, intrabronchially, transdermally, topically, intravaginally, or rectally.

The compounds of this invention are particularly advantageous as immunosuppressive, antiinflammatory, antifungal, antiproliferative, and antitumor agents because of their water solubility.

It is contemplated that when the compounds of this invention are used as an immunosuppressive or antiinflammatory agent, they can be administered in conjunction with one or more other immunoregulatory agents. Such other immunoregulatory agents include, but are not limited to azathioprine, corticosteroids, such as prednisone and methylprednisolone, cyclophosphamide, rapamycin, cyclosporin A, FK-506, OKT-3, and ATG. By combining the compounds of this invention with such other drugs or agents for inducing immunosuppression or treating inflammatory conditions, the lesser amounts of each of the agents are required to achieve the desired effect. The basis for such combination therapy was established by Stepkowski whose results showed that the use of a combination of rapamycin and cyclosporin A at subtherapeutic doses significantly prolonged heart allograft survival time. [Transplantation Proc. 23: 507 (1991)].

This invention also comprises pharmaceutical compositions comprising one or more of the PEG esters of this invention and one or more pharmaceutically acceptable carriers or excipients. The pharmaceutical carriers or excipients may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

The compounds of this invention may be administered rectally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semi-permeable membrane covering a reservoir containing the active ingredient with or without a carrier, or a matrix containing the active ingredient. Other occlusive devices are known in the literature.

In addition, the compounds of this invention may be employed as a solution, cream, or lotion by formulation with pharmaceutically acceptable vehicles containing 0.1 - 5 percent, preferably 2%, of active compound which may be administered to a fungally affected area.

The dosage requirements vary with the particular compositions employed, the route of administration, the severity of the symptoms presented and the particular subject being treated. Based on the results obtained in the standard pharmacological test procedures, projected daily dosages of active compound would be 0.1 µg/kg - 100 mg/kg, preferably between 0.001 - 25 mg/kg, and more preferably between 0.01 - 5 mg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

The 42-O-(2-Hydroxy)ethyl on SDZ-RAD esterified compound of this invention can be prepared by initially acylating the hydroxyl group on 42-O-(2-Hydroxy)ethyl or 31-position or both position of SDZ-RAD with an acylating agent having the general structure X-CH₂CO₂H, where X is a suitable leaving group such as iodine or bromine, in the presence of a coupling reagent, such as dicyclohexylcarbodiimide (DCC) and a base catalyst such as dimethylaminopyridine (DMAP) to provide either a 42-O-(2-Hydroxy)ethyl and/or 31-hydroxy acylated SDZ-RAD having the following structure (II) where R¹ and R² are independently from -H and -COCH₂X.

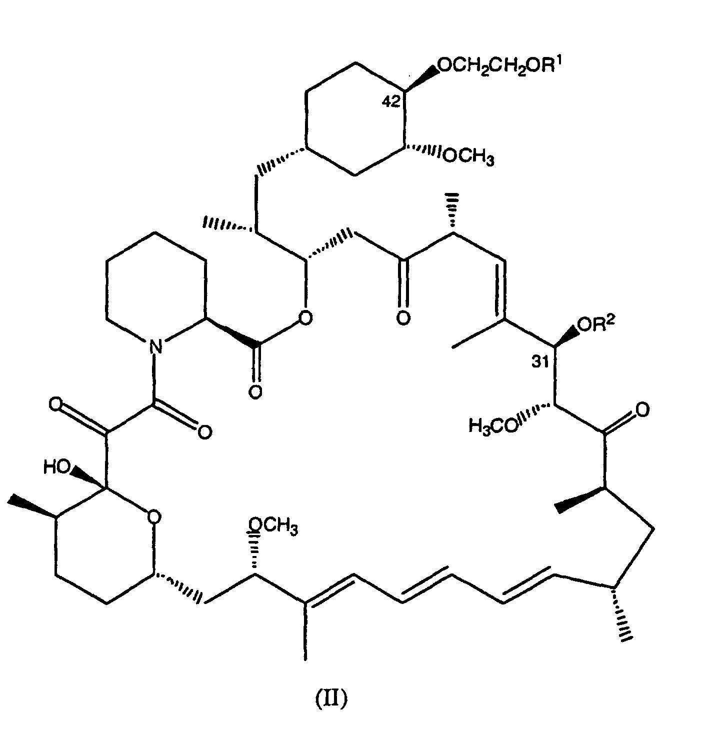

Every possible compound can be separated by chromatography. Reaction of the acylated SDZ-RAD with monomethoxypoly(ethylene glycol) thiol in the presence of base such as sodium bicarbonate provides the desired ester on 42-O-(2-hydroxy)ethyl of SDZ-RAD of this invention. This novel method can be applied to any hydroxyalkyl, dihydroxyalkyl, hydroalkylarylalkyl, dihydroalkylarylalkyl, dihydroxyalkylally derivative of rapamycin in U.S. patent 5,655,772.

Accordingly this invention provides a process for preparing a compound of formula I as defined above which comprises reacting a compound of formula II as shown above wherein R¹ is COCH₂X where X is a suitable leaving group such as iodine or bromine and R² is hydrogen, with a compound of formula (III)

HS CH₂CH₂(OCH₂CH₂)ₙOCH₃ (III)

wherein n is as defined in connection with formula I.

The preparation of rapamycin 42-silyl ethers is described in U.S. Patent B1 5,120,842, which is hereby incorporated by reference. In the case of the tert-butyl dimethylsilyl protecting group, deprotection can be accomplished under mildly acidic conditions, such as acetic acid / water / THF. The deprotection procedure is described in Example 15 of U.S. Patent 5,118,678, which is hereby incorporated by reference.

This invention also covers analogous esters of other rapamycins known in the art such as, but not limited to, 29-demethoxyrapamycin, [U.S. Patent 4,375,464, 32-demethoxyrapamycin under C.A. nomenclature]; rapamycin derivatives in which the double bonds in the 1-, 3-, and/or 5-positions have been reduced [U.S. Patent 5,023,262]; 29-desmethylrapamycin [U.S. Patent 5,093,339, 32-desmethylrapamycin under C.A. nomenclature]; 7,29-bisdesmethylrapamycin [U.S. Patent 5,093,338, 7,32-desmethylrapamycin under C.A. nomenclature]; 27-hydroxyrapamycin [U.S. Patent 5,256,790] and 15-hydroxyrapamycin [U.S. Patent 5,102,876]. This invention also covers esters at the 31-position of 42-oxorapamycin [U.S. Patent 5,023,263]. The disclosures in the above cited U.S. Patents are hereby incorporated by reference.

The reagents used in the preparation of the compounds of this invention can be either commercially obtained or can be prepared by standard procedures described in the literature.

The following examples illustrate the preparation and biological activities of representative compounds of this invention.

### Experimental Section

### Material and Instruments

1,3-Dicyclohexylcarbodiimide (DCC) and 4-dimethylaminopyridine were purchased from Aldrich Chemical Co. (Milwaukee, WI). Methoxy-PEG-SH of average molecular weight 5000 (mPEG-SH 5000) was purchased from Shearwater Polymers, Inc. (Huntsville, Al). SDZ-RAD was obtained from Chemical Development Wyeth-Ayerst Research, Pearl River, NY. All solvents were HPLC grade and all other chemicals were analytical reagent or equivalent. The preparative HPLC consisted of two Dynamax solvent delivery systems (Model SD-1) and one Dynamax absorbance detector (Model UV-1) from Rainin Instrument Inc.(Woburn, MA). An automatic speed-vac concentrator (Savant, Model AS 160) was from Savant Instruments, Inc. (Holbrook, NY) and a BUCHI rotary evaporation system (RE 260 and R 124) was from Buchi (Flawil, Switzerland). ¹H-NMR spectra were recorded on 400 MHz NMR spectrophotometer using CDCl₃ as solvents. Mass spectra were obtained from API 365 mass spectrophotometer or MALDI/TOF mass spectrophotometer from PE Sciex. All samples were prepared and run at ambient temperature.

### HPLC Method

The analytical HPLC system consisted of a Hewlett-Packard model 1090 LC with a 1040 diode array detector system. A *µ*-Bondapak C-18 (300 x 3.9 mm) column from Waters was used. The mobile phase A was 10% acetonitrile, 90% 0.1 M 0.1 M tetraethylammonium acetate (TEAA) buffer at pH 4.5. The mobile phase B was 90% acetonitrile, 10% 0.1 M TEAA buffer at pH 4.5. The gradient was from 50% B to 100% B in 30 min then held 100% B for 5 min before going beck original 50% B. The column was equilibrated at 50% B for 15 min before next injection. The column temperature was 40 °C and flow rate was 1 mL/min. The detection wavelength was set at 280 nm. The injection volume was 10 µl.

### Preparation of SDZ-RAD-iodoacetate ester

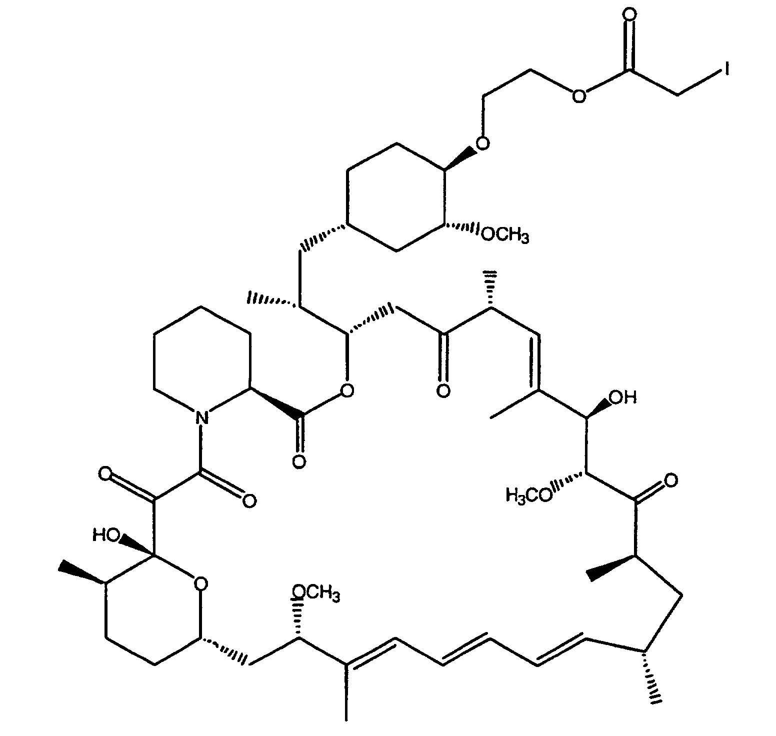

SDZ-RAD (0.50 g 5.2 x 10⁴ mole), 4-Dimethylaminopyridine (3.0 mg) and 1,3-Dicyclohexylcarbodiimide (0.136 g, 6.6 x 10⁻⁴ mole) were dissolved in 20 ml anhydrous methylene chloride in a 150 ml round-bottom flask. Iodoacetic acid (0.116 g, 6.3 x 10⁻⁴ mole) was dissolved in 10 ml anhydrous methylene chloride. The iodoacetic acid solution was added into the reaction mixture over a period of 10 min. with stirring by a magnetic bar. Then the reaction mixture was stirred at room temperature for another 2.5 h. The solution was then filtered through a sintered glass filter. The filtrate was transferred to a separatory funnel, washed 50 ml of sodium bicarbonate solution (5.5 g/ 100 ml) and then washed with 2 x 50 ml of water. The methylene chloride layer was dried with 5g anhydrous sodium sulfate for 4 hours. Then sodium sulfate was filtered out and methylene chloride was removed by rotary evaporation. A total of 0.60g pale yellow solid was obtained. Isolation of pure SDZ-RAD-iodoacetate ester was performed by preparative HPLC on a Prep Nova-pak HR C18 (300 x 19 mm) column from Waters. SDZ-RAD-iodoacetate ester eluted at 18.4 min using a gradient ( 30% A, 70% B for 5 min. then to 100% B in 30 min.). A is 90% water, 10% acetonitrile; B is 10% water, 90% acetonitrile. The fraction was collected and extracted by 2 x 50 ml methylene chloride. The organic layer was combined and dried with anhydrous sodium sulfate for 4 h. The organic solvent was removed by rotary evaporation to dryness. A yellowish solid was obtained (0.102 g). ¹H NMR (CDCL₃, 400 MHz) d 3.72 (s, 2H, I-CH₂-CO₂-), 4.28 (m, 2H, -CO₂-CH₂-). MS m/z 1134.6 (M+NH₄)⁺; m/z 1184.6 (M+OAC)⁻. MS/MS 213.0 (ICH₂CO₂CH₂CH₂)⁺.

### Preparation of SDZ-RAD-PEG SH 5000 conjugate

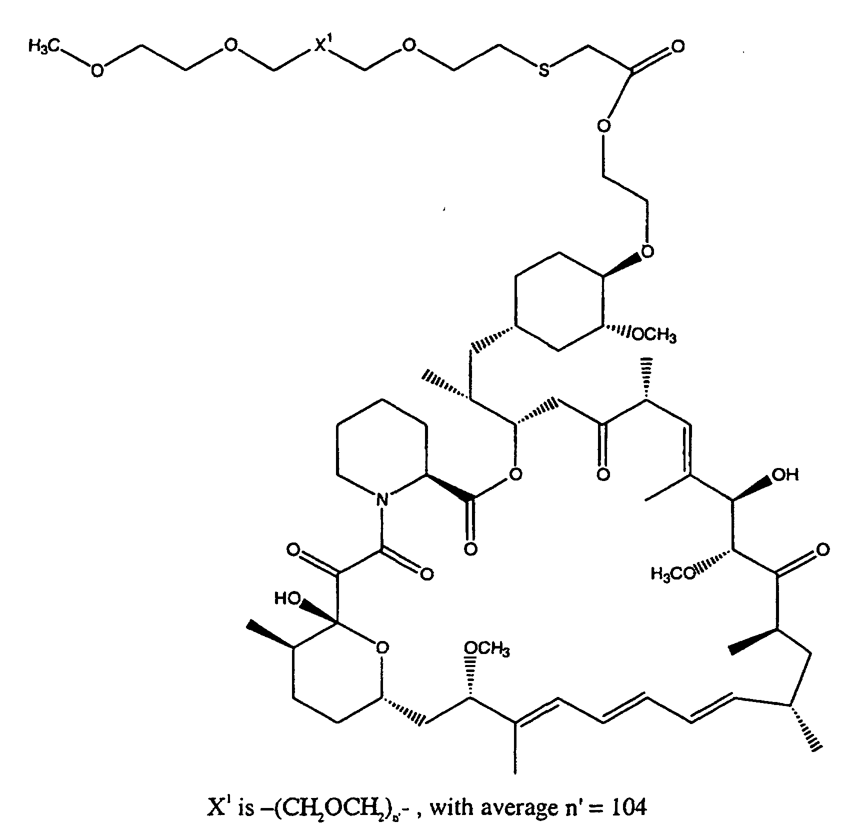

SDZ-RAD iodoacetate ester (76 mg, 6.8 x 10⁻⁵mole) was dissolved in 30 ml of solution containing 50% acetonitrile and 50% aqueous NaHCO₃ (0.1 M) solution. The solution was flushed with N₂ for 10 min. The original sample 10 µL was taken for HPLC analysis. Then mPEG-SH 5000 (337 mg, 6.8 x 10⁻⁵ mole) was added to the reaction solution and the reaction mixture was stirred at room temperature for another 30 min. The reaction was checked again by taking 10 µL sample for HPLC analysis. The chromatogram showed that SDZ-RAD iodoacetate ester was 100% converted to SDZ-RAD-PEG conjugate. The reaction mixture was extracted with 2 x 150 ml methylene chloride. The organic layer was dried with anhydrous sodium sulfate then filtered. The filtrate was concentrated to a volume of 20 ml by rotary evaporation. The crude product was precipitated out after adding 200 mL ether. A total of 340 mg pale yellow powder was obtained after filtered out by a sintered glass funnel and dried under vacuum. Isolation of pure SDZ-RAD-PEG conjugate, which may also be referred to as pegylated SDZ-RAD, was performed by preparative HPLC on a Prep Nova-pak HR C18 (300 x 19 mm) column from Waters. SDZ-RAD-iodoacetate ester eluted at 15 min. using a gradient (60% A, 40% B for 5 min. then at 20% A, 80% B in 30 min.) The fraction was collected and extracted by 2 x 100 ml methylene chloride. The organic layer was combined and dried with anhydrous sodium sulfate for 4 hr. The organic solvent was removed by rotary evaporation to dryness. The residue was dissolved in 5 ml methylene chloride and was precipitated out after adding 150 ml ether. A pale yellow powder was obtained after filtered out by a sintered glass funnel and dried under vacuum. ¹H NMR (CDCl₃, 400 MHz) δ 2.84 (t, 2H, S-CH₂-CH₂), 3.31 (s, 2H, CO-CH₂-S), 3.38 (s, 3H, -OCH₃), 4.26 (m, 2H, -CO₂-CH₂-). MS (MALD/TOF) m/z 5795.5.

### U87MG Human Glioblastoma (ATCC # HTB-14)

### 3H Thymidine Incorporation Protocol

**Growth Medium:**
   BRL Minimum Essential Medium with Earle Salts (500 ml)
   + 5mLBRL MEM Non-Essential Amino Acids (10mM)
   +5 mLBRL Penicillin-Streptomycin
   (10000u/ml,10000ug/ml)
   +5mLBRL Na Pyruvate Solution (100mM)
   +5mLBRL L-Glutamine 200mM
   +50mLBRL Fetal Bovine Serum (Qualified)
**Assay:**
   1. Cells were trypsinized and plated at a concentration of 10⁴ cells/well in a final volume of 200 *µ*l growth medium in 96-well flat bottom plates and allowed to adhere for 24 hours at 37°C.
   2. The media was removed by aspiration with care to not disturb the cell monolayer. 200 *µ*l of fresh growth media was added per well, allowing enough wells for samples to be run in triplicate. Compounds were added in 10 *µ*l PBS solutions and incubated for another 48 hours at 37°C.
   3. During the last 5 hours of incubation, plates were labeled with 1*µ*Ci 3H thymidine per well. (NEN thymidine, catalog # NET-027, 6.7 Ci/mmole). The 1*µ*Ci was added in 10 *µ*l of PBS (on the day of harvest.). The plates were returned to the incubator for the last 5 hours.
   4. The radioactive media was removed by aspiration, with care not to disturb the cell monolayer. 50 *µ*l of BRL 10X Trypsin was added to each well, followed by incubation at 37°C for 10 minutes or until the monolayer was loosened from the well bottom. Samples were harvested on a glass fiber filter mat using a Skatron 96 well harvester. Mats were counted in a Wallac Betaplate counter.

### Results

| Compound | IC₅₀ |
|---|---|
| Rapamycin | 0.5 ng/mL |
| SDZ-RAD | 2.0 ng/mL |
| SDZ-RAD-PEG 5000 conjugate | 0.5 ng/mL* |

| | |
|---|---|
| * molar equivalent to SDZ-RAD | |

## Claims

1. A compound of the structure (I) 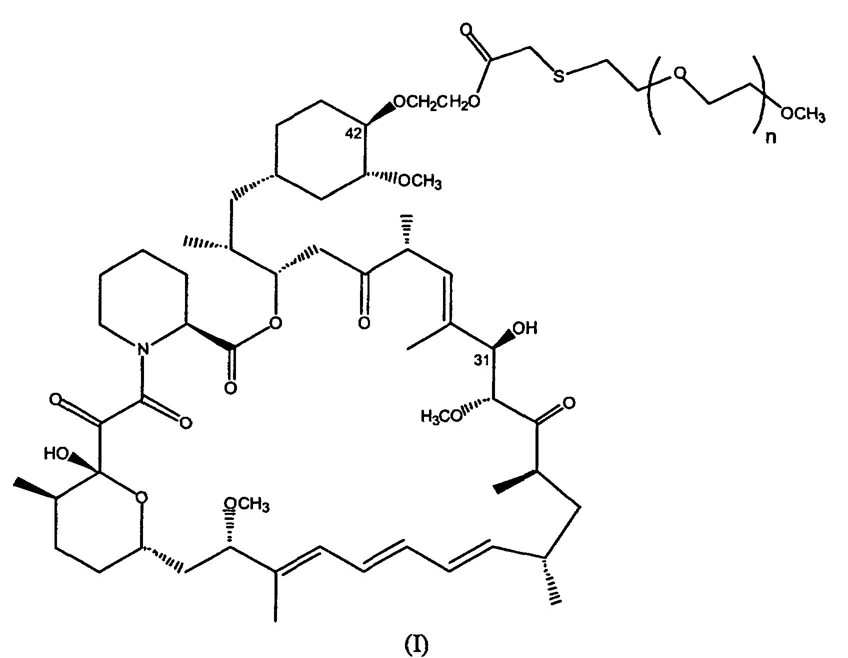 wherein n is an integer from 5 - 450.

2. The compound of claim 1 wherein n = 5 - 200.

3. The compound of claim 1 wherein n = 8 - 135.

4. The compound of claim 1 wherein n = 8-20.

5. The compound of claim 1 wherein n = 90 - 120.

6. The compound of claim 1 which is SDZ-RAD-PEG SH 5000 conjugate.

7. A process for preparing a compound of formula (I) as claimed in claim 1 which comprises reacting a compound of formula (II) 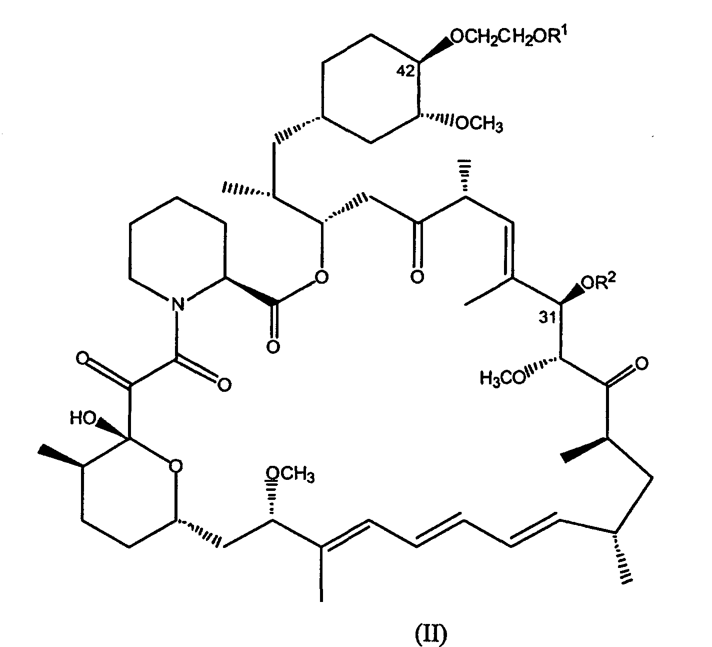 wherein R¹ is COCH₂X where X is a suitable leaving group and R² is hydrogen with a compound of formula:
HS CH₂CH₂(OCH₂CH₂)ₙOCH₃
wherein n is as defined in connection with formula I.

8. Use of a compound of any one of Claims 1 to 6 for the preparation of a medicament for treating transplantation rejection or graft vs. host disease in a mammal.

9. Use of a compound of any one of Claims 1 to 6 for the preparation of a medicament for treating a fungal infection in a mammal.

10. Use of a compound of any one of Claims 1 to 6 for the preparation of a medicament for treating rheumatoid arthritis in a mammal.

11. Use of a compound of any one of Claims 1 to 6 for the preparation of a medicament for treating restenosis in a mammal.

12. Use of a compound of any one of Claims 1 to 6 for the preparation of a medicament for treating pulmonary inflammation in a mammal.

13. Use of a compound of any one of Claims 1 to 6 for the preparation of a medicament for treating solid tumors in a mammal.

14. A use according to claim 13, wherein the solid tumor is a carcinoma or sarcoma.

15. A pharmaceutical composition which comprises a compound of the formula (I) as claimed in any one of claims 1 to 4 and a pharmaceutically acceptable carrier or excipient.

16. The pharmaceutical composition of claim 15 in which the compound of formula (I) is SDZ-RAD-PEG SH5000 conjugate.

## Patentansprüche

1. Verbindung der Struktur (I), 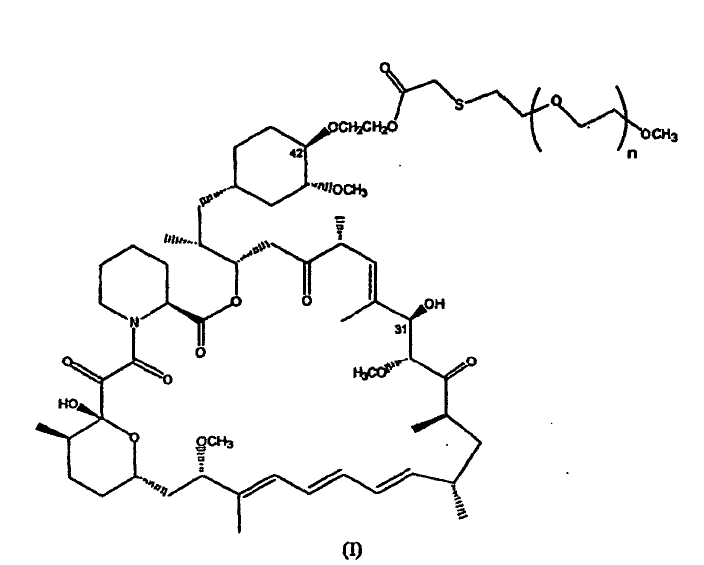 worin n eine ganze Zahl von 5-450 ist.

2. Verbindung nach Anspruch 1, worin n = 5-200 ist.

3. Verbindung nach Anspruch 1, worin n = 8-135 ist.

4. Verbindung nach Anspruch 1, worin n = 8-20 ist.

5. Verbindung nach Anspruch 1, worin n = 90-120 ist.

6. Verbindung nach Anspruch 1, welche SDZ-RAD-PEG SH 5000 Konjugat ist.

7. Verfahren zum Herstellen einer Verbindung der Formel (I), wie in Anspruch 1 beansprucht, welches umfasst: Umsetzen einer Verbindung der Formel (II) 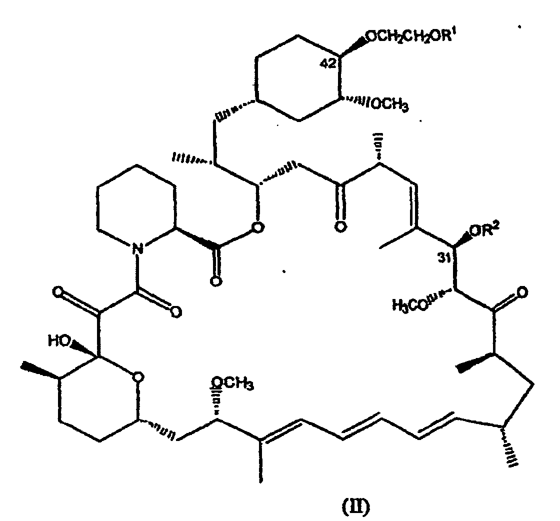 worin R¹ für COCH₂X steht, worin X eine geeignete Abgangsgruppe darstellt, und R² für Wasserstoff steht, mit einer Verbindung der Formel:
HSCH₂CH₂ (OCH₂CH₂)ₙ OCH₃,
worin n wie in Verbindung mit Formel 1 definiert ist.

8. Verwendung einer Verbindung nach einem der Ansprüche 1 bis 6 zur Herstellung eines Medikaments zum Behandeln von Transplantationsabstoßung oder Transplantat-gegen-Wirt-Erkrankung in einem Säuger.

9. Verwendung einer Verbindung nach einem der Ansprüche 1 bis 6 zur Herstellung eines Medikaments zum Behandeln einer Pilzinfektion in einem Säuger.

10. Verwendung einer Verbindung nach einem der Ansprüche 1 bis 6 zur Herstellung eines Medikaments zum Behandeln von Rheumatoidarthritis in einem Säuger.

11. Verwenden einer Verbindung nach einem der Ansprüche 1 bis 6 zur Herstellung eines Medikaments zum Behandeln von Restenose in einem Säuger.

12. Verwendung einer Verbindung nach einem der Ansprüche 1 bis 6 zur Herstellung eines Medikaments zum Behandeln von pulmonarer Entzündung in einem Säuger.

13. Verwendung einer Verbindung nach einem der Ansprüche 1 bis 6 zur Herstellung eines Medikaments zum Behandeln von festen Tumoren in einem Säuger.

14. Verwendung gemäß Anspruch 13, wobei der feste Tumor ein Karzinom oder Sarkom ist.

15. Pharmazeutische Zusammensetzung, welche eine Verbindung der Formel (I) , wie in einem der Ansprüche 1 bis 4 beansprucht, und einen pharmazeutisch annehmbaren Träger oder Exzipienten umfasst.

16. Pharmazeutische Zusammensetzung nach Anspruch 15, wobei die Verbindung der Formel (I) SDZ-RAD-PEG SH5000 Konjugat ist.

## Revendications

1. Composé de structure (I) 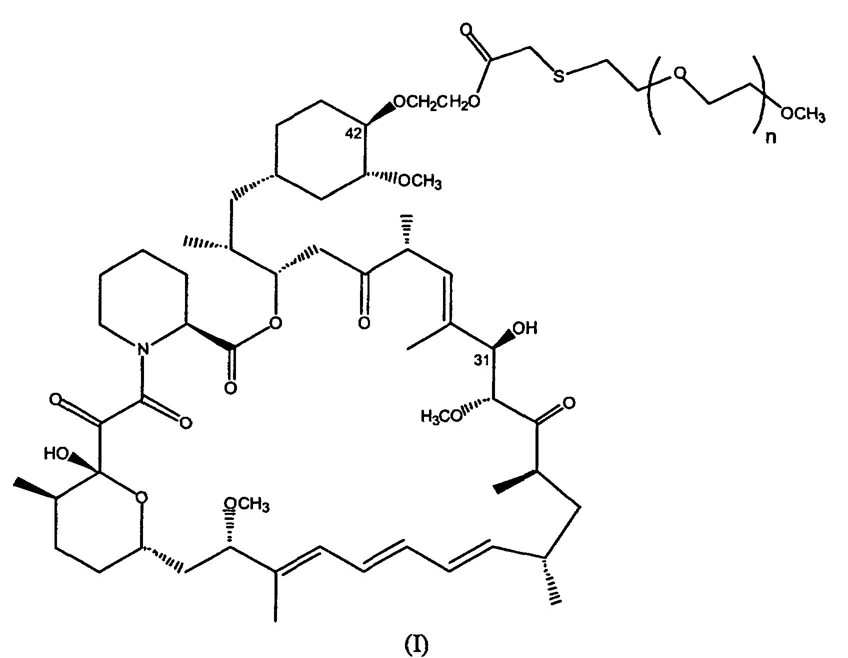 dans laquelle
n est un nombre entier de 5 à 450.

2. Composé selon la revendication 1 dans lequel n = 5 - 200.

3. Composé selon la revendication 1 dans lequel n = 8 - 135.

4. Composé selon la revendication 1 dans lequel n = 8 - 20.

5. Composé selon la revendication 1 dans lequel n = 90 - 120.

6. Composé selon la revendication 1 qui est un conjugué SDZ-RAD-PEG SH 5000.

7. Procédé pour la préparation d'un composé de formule (I) tel que revendiqué dans la revendication 1, qui consiste à faire réagir un composé de formule (II) 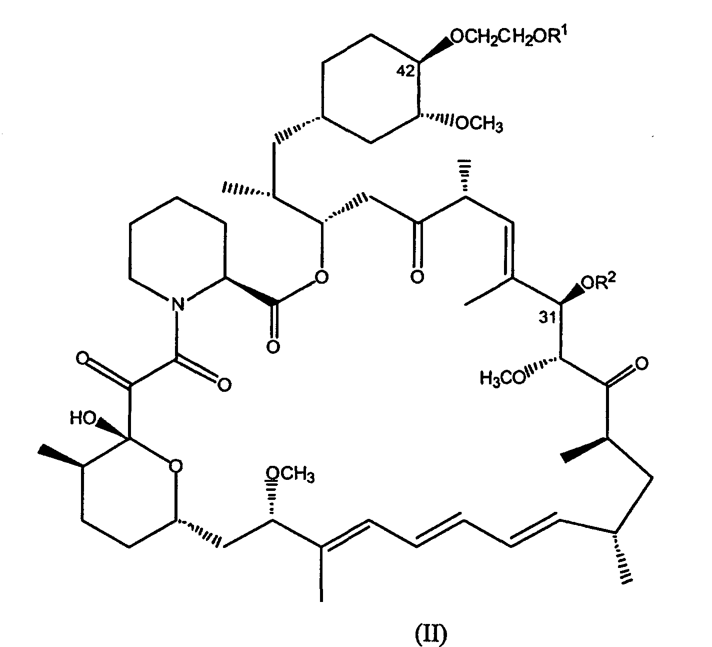 dans laquelle R¹ est COCH₂X, où X est un groupe partant approprié et R² est un hydrogène, avec un composé de formule:
HSCH₂CH₂ (OCH₂CH₂)ₙ OCH₃,
dans laquelle n est comme défini en rapport avec la formule (I).

8. Utilisation d'un composé selon l'une quelconque des revendications 1 à 6 pour la préparation d'un médicament pour le traitement d'un rejet de transplantation ou d'une réaction du greffon contre l'hôte chez un mammifère.

9. Utilisation d'un composé selon l'une quelconque des revendications 1 à 6 pour la préparation d'un médicament pour le traitement d'une infection provoquée par un champignon chez un mammifère.

10. Utilisation d'un composé selon l'une quelconque des revendications 1 à 6 pour la préparation d'un médicament pour le traitement de l'arthrite rhumatoïde chez un mammifère.

11. Utilisation d'un composé selon l'une quelconque des revendications 1 à 6 pour la préparation d'un médicament pour le traitement d'une resténose chez un mammifère.

12. Utilisation d'un composé selon l'une quelconque des revendications 1 à 6 pour la préparation d'un médicament pour le traitement d'une inflammation pulmonaire chez un mammifère.

13. Utilisation d'un composé selon l'une quelconque des revendications 1 à 6 pour la préparation d'un médicament pour le traitement de tumeurs solides chez un mammifère.

14. Utilisation selon la revendication 13 dans laquelle la tumeur solide est un carcinome ou un sarcome.

15. Composition pharmaceutique qui comprend un composé de formule (I) tel que revendiqué dans l'une quelconque des revendications 1 à 4 et un vecteur ou un excipient pharmaceutiquement acceptables.

16. Composition pharmaceutique selon la revendication 15 dans laquelle le composé de formule (I) est un conjugué SDZ-RAD-PEG SH 5000.
